# EUROPEAN PATENT SPECIFICATION

(11) **EP 0 487 613 B1**
(45) Date of publication and mention of the grant of the patent: **30.10.1996**
(21) Application number: 90913091.6
(22) Date of filing: 07.08.1990
(51) Int. Cl.: C07K 14/00, C12N 15/27, A61K 38/00

(54) **A MEGAKARYOCYTOPOIETIC FACTOR**
MEGAKARYOCYTOPOIETISCHER FAKTOR
FACTEUR MEGAKARYOCYTOPOIETIQUE

(30) Priority: 08.08.1989 US 390901; 28.12.1989 US 457196; 29.06.1990 US 546114
(43) Date of publication of application: 03.06.1992
(60) Divisional application: 95119320.0
(73) Proprietor: GENETICS INSTITUTE, INC., Cambridge, Massachusetts 02140 (US)
(72) Inventor: GESNER, Thomas, G., Beverly, MA 01915 (US); CLARK, Steven, C., Winchester, MA 01890 (US); TURNER, Katherine, Melrose, MA 02176 (US); HEWICK, Rodney, M., Lexington, MA 02173 (US)
(74) Representative: VOSSIUS & PARTNER
(86) International application number: US9004421
(87) International publication number: WO9102001

(56) References cited:
- JOURNAL OF CLINICAL INVESTIGATION, vol. 75, April 1985, American Society for Clinical Investigation Inc.; R. HOFFMAN et al., pp. 1174-1182
- CHEMICAL ABSTRACTS, vol. 105, no. 5, Columbus, OH (US); H.H. YANG et al., AN 36292x
- CHEMICAL ABSTRACTS, vol. 105, no. 13, Columbus, OH (US); M. KAWAKITA et al., AN 109405y
- CHEMICAL ABSTRACTS, vol. 98, no. 11, Columbus, OH (US); T. MIYAKE et al., AN 83667v
- EXPERIMENTAL HEMATOLOGY, vol. 15, no. 5, 1987, p. 492
- EXPERIMENTAL HEMATOLOGY, vol. 14, no. 6, 1986, p. 490

## Description

The present invention relates generally to a novel protein factor which is important in regulating the human hematopoietic system. More specifically the invention discloses a novel protein factor that stimulates megakaryocytic colony formation and the differentiation or maturation of megakaryocyte progenitors. Also provided are processes for obtaining the factor in homogeneous form.

### Background of the Invention

Megakaryocytes are the hematopoietic cells, largely found in the bone marrow, but also in peripheral blood and perhaps other tissues as well, which produce platelets (also known as thrombocytes) and subsequently release them into circulation. Megakaryocytes, like all of the hematopoietic cells of the human hematopoietic system, ultimately derive from a primitive stem cell after passing through a complex pathway comprising many cellular divisions and considerable differentiation and maturation.

The platelets derived from these megakaryocytic cells are critical for initiating blood clot formation at the site of injury. Platelets also release growth factors at the site of clot formation that speed the process of wound healing and may serve other functions. However, in patients suffering from depressed levels of platelets (thrombocytopenia) the inability to form clots is the most immediate and serious consequence, a potentially fatal complication of many therapies for cancer. Such cancer patients are generally treated for this problem with platelet transfusions. Other patients frequently requiring platelet transfusions are those undergoing bone marrow transplantation or patients with aplastic anemia.

Platelets for such procedures are obtained by plateletphoresis from normal donors. Like most human blood products, platelets for transfusion have a relatively short shelf-life and also expose the patients to considerable risk of exposure to dangerous viruses, such as the human immunodeficiency virus (HIV).

Clearly the ability to stimulate endogenous platelet formation in thrombocytopenic patients with a concomitant reduction in their dependence on platelet transfusion would be of great benefit. In addition the ability to correct or prevent thrombocytopenia in patients undergoing radiation therapy or chemotherapy for cancer would make such treatments safer and possibly permit increases in the intensity of the therapy thereby yielding greater anti-cancer effects.

For these reasons considerable research has been devoted to the identification and purification of factors involved in the regulation of megakaryocyte and platelet production. Although there is considerable controversy, the factors regulating the growth and differentiation of hematopoietic cells into mature megakaryocytes and the subsequent production of platelets by these cells are believed to fall into two classes: (1) megakaryocyte colony-stimulating factors (meg-CSFs) which support the proliferation and differentiation of megakaryocytic progenitors in culture, and (2) thrombopoietic (TPO) factors which support the differentiation and maturation of megakaryocytes resulting in the production and release of platelets. [See, e.g., E. Mazur, Exp. Hematol., 15:340-350 (1987).]

Either class of factors is defined by bioassay. Factors with meg-CSF activity support megakaryocyte colony formation, while factors with TPO activity elicit an elevation in the numbers of circulating platelets when administered to animals. It is not clear how many species of factors exist that have either or both of these activities. For example, human IL-3 supports human megakaryocyte colony formation and, at least in monkeys, also frequently elicits an elevation in platelet count However, IL-3 influences hematopoietic cell development in all of the hematopoietic lineages and can be distinguished from specific regulators of megakaryocytopoiesis and platelet formation which interact selectively with cells of the megakaryocytic lineage.

From the studies reported to date, it is not clear whether activities identified as meg-CSF also have TPO activity or vice versa. Many different reports in the literature describe factors which interact with cells of the megakaryocytic lineage. Several putative meg-CSF compositions have been derived from serum [See, e.g., R. Hoffman et al, J. Clin. Invest., 75:1174-1182 (1985); J. E. Straneva et al, Exp. Hematol., 15:657-663 (1987); E. Mazur et al, Exp. Hematol., 13:1164-1172 (1985]. A larger number of reports of a TPO factor are in the art. [See, e.g., T. P. McDonald, Exp. Hematol., 16:201-205 (1988); T. P. McDonald et al, Biochem. Med. Metab. Biol., 37:335-343 (1987); T. Tayrien et al, J. Biol. Chem., 262: 3262-3268 (1987) and others].

Although there have been numerous additional reports tentatively identifying such regulatory factors, the biochemical and biological identification and characterization of these factors has been hampered by the small quantities of the naturally occurring factors available from natural sources, e.g., blood and urine. At present there is no identification of a single purified factor useful as a meg-CSF or TPO for pharmaceutical use in replacing serum-derived products or platelets.

There remains a need in the art for additional proteins purified from their natural sources or otherwise produced in homogeneous form, which are capable of stimulating or enhancing the production of platelets in vivo, to replace presently employed platelet transfusions.

### Brief Summary of the Invention

In one aspect the present invention provides a novel human megakaryocytopoietic factor (meg-CSF) which is substantially free from other human proteins. This protein may be purified from cell sources producing the factor naturally or upon induction with other factors. meg-CSF may also be synthesized by chemical techniques, or a combination of the above-listed techniques.

The meg-CSF of the present invention has been found to stimulate the growth and development of colonies consisting of intermediate and large size megakaryocytes in an assay using murine bone marrow target cells. meg-CSF displays biological activity in this assay of greater than 5X10⁷ dilution units per milligram of protein. meg-CSF has also displayed activity in an assay using human cells, as described in Example 8 below.

Active meg-CSF has an apparent molecular weight of approximately 28-38 kd as determined by sodium dodecyl sulfate polyacrylamide gel electrophoresis under non-reducing conditions. meg-CSF has an apparent molecular weight of approximately 20-27 kd as determined by sodium dodecyl sulfate polyacrylamide gel electrophoresis under reducing conditions.

Meg-CSF is also characterized by the four amino acid sequences or fragments thereof, recited below as sequences (a) through (d).

A further aspect of the present invention is a process for isolating and purifying the meg-CSF composition of the present invention or a fragment thereof from human urine. This purification process provided by the present invention involves the steps of concentrating the urine; subjecting it to anion exchange column chromatography; followed by cation exchange column chromatography; subjecting the resulting materials to lectin affinity chromatography followed by cation exchange fine performance liquid chromatography (FPLC) and three elutions through reverse phase high pressure liquid chromatography (HPLC) using different solvent solutions for each HPLC run.

A further aspect of the present invention is homogeneous meg-CSF, purified from urine or produced via synthetic techniques, which is characterized by a specific activity in the murine fibrin clot assay of greater than 5X10⁷ dilution units/mg.

Another aspect of this invention provides pharmaceutical compositions containing a therapeutically effective amount of homogeneous meg-CSF or an effective amount of one or more active peptide fragments thereof. These pharmaceutical compositions may be employed for treating disease states or disorders characterized by a deficiency of platelets. Thus the meg-CSF composition of the present invention or pharmaceutically effective fragments thereof may be employed in the treatment of aplastic anemias resulting from chemotherapy or thrombocytopenia. meg-CSF may be used as an adjunctive therapy for bone marrow transplant patients.

These meg-CSF compositions may additionally comprise an effective amount of at least one other TPO-like factor, a cytokine, hematopoietin, interleukin, growth factor, or antibody. Suitable cytokines are, for example, G-CSF, CSF-1, GM-CSF, IL-1, IL-3, IL-4, erythropoietin, IL-11, IL-6, TPO, M-CSF and IL-7.

Still another aspect of the present invention are antibodies directed against human meg-CSF or a fragment thereof. As part of this aspect, therefore, the invention claims cell lines capable of secreting such antibodies and methods for their production and use in diagnostic or therapeutic procedures.

Other aspects and advantages of the present invention will be apparent upon consideration of the following detailed description of preferred embodiments thereof.

### Detailed Description of the Invention

The novel human megakaryocyte colony stimulating factor, meg-CSF, provided by the present invention is a homogeneous protein or proteinaceous composition substantially free of association with other human proteinaceous materials. This protein can be obtained as a homogeneous protein purified from human urine or a mammalian cell line secreting or expressing it. Further meg-CSF or active fragments thereof may be chemically synthesised.

meg-CSF of the present invention is characterised by the following biochemical and biological properties:

It is substantially free from association with other proteinaceous material and further characterised by the following characteristics:
(a) not digestible with N-glycanase under standard conditions;
(b) an apparent molecular weight of about 28-38 kD as determined by 12% sodium dodecyl sulfate polyacrylamide gel electrophoresis under non-reducing conditions;
(c) an apparent molecular weight of about 20-27 kD as determined by 12% sodium dodecyl sulfate polyacrylamide gel electrophoresis under reducing conditions; and
(d) the partial amino acid sequences: (i)    Ser Arg Cys Phe Glu Ser Phe Glu Arg; 
(ii) Arg Val Cys Thr Ala Glu Leu Ser Cys Lys
Gly;
 
(iii) Lys Ala Pro Pro Pro (X) Gly Ala Ser Gln
Thr Ile Lys; and
 
(iv)    Lys Tyr Asp Lys Cys Cys Pro Asp Tyr Glu
        Ser Phe Cys Ala Glu Val His Asn Pro,
 wherein (X) = Ser or Thr. In the sequences, (X) indicates that the residue is not yet absolutely identified, but may be Ser or Thr; and () indicates tentative identification of a residue. The sequences identified above as (a) through (d) were originally determined from purified material from step 8, the third HPLC purification step of the purification procedure, omitting step 7. The same sequences also have been obtained from the material, when purified through all eight steps.
In a preferred embodiment, the meg-CSF of the invention is further characterised by possessing a specific activity of between approximately 5 X 10⁷ and 5 X 10⁸ dilution units/mg protein in a megakaryocyte colony formation assay, or characterised biologically by a specific activity in a murine fibrin clot megakaryocyte colony formation assay of 2 X 10⁸ dilution units/mg protein.

In a further preferred embodiment, the meg-CSF of the invention has the ability to stimulate growth and development of colonies consisting of intermediate and large sized megakaryocyte cells.

In a still further preferred embodiment, the protein of the present invention is further characterised by one or more of the following characteristics:
(1) the ability to bind SP-Zeta Prep under acidic conditions of pH 4.5;
(2) the ability to bind to Wheat Germ-Sepharose and Concanavalin-A Sepharose;
(3) the ability to elute between 23-33% acetonitrile on a reverse-phase HPLC (C4) column in a solvent of acetonitrile in trifluoroacetic acid;
(4) the ability to elute between 6-15% n-propanol on a reverse-phase HPLC (C18) column in a solvent of n-propanol in pyridine and acetic acid;
(5) the ability to elute between 27-37% acetonitrile on a reverse-phase HPLC (C4) column in a solvent of heptafluorobutyric acid in acetonitrile.

The biological activity of the meg-CSF composition of the present invention is demonstrated by its ability to stimulate the growth and development of colonies consisting of intermediate and large size megakaryocytes in culture. In the murine fibrin clot megakaryocyte colony formation assay, the meg-CSF composition of the present invention stimulates colonies of an average of 3-6 megakaryocytes. In the murine agar meg-CSF assay, the meg-CSF composition of the present invention stimulates colonies of megakaryocytes. The meg-CSF composition of the present invention has inconsistently shown activity in the human plasma clot megakaryocyte colony formation assay.

meg-CSF was originally detected in the urine of human patients with bone marrow transplants. These patients demonstrate an enhanced level of meg-CSF activity. Human meg-CSF was initially purified from this human urine by a sequence of purification steps and techniques specifically described in Example 1 below. However, this factor may also be purified from other sources, e.g., human cell lines.

The purification techniques employed in obtaining meg-CSF from the human urine comprises the following steps. The purification steps include concentrating pooled bone marrow transplant patient urine through an Amicon YM-10 filter. The concentrated urine is passed through an anion exchange chromatographic column and the flow-through is bound onto a cation exchange chromatographic column. The urinary protein eluate was then subjected to pooling, dialyzing and heating and applying it to a lectin affinity chromatographic column. This eluate is then dialyzed and applied to a cation exchange FPLC column. Finally this eluate is applied through three cycles of reverse phase HPLC using different solvent systems.

Batches with the highest levels of meg-CSF in the murine fibrin clot assay, described below, were selected for further purification at the semi-preparative scale (between 30 and 100 liters urine equivalent) to maximize recovery and yield.

Thus the homogeneous meg-CSF may be obtained by applying the above purification procedures, which are described in detail in Example 1, to human urine or other sources of human meg-CSF, e.g., activated peripheral blood leukocytes and human placenta. Other tissue sources and cell lines such as C10-MJ2 (an HTLV1-transformed T cell line) and HEK (primary human embryonic kidney cells) may also be sources of this protein. Procedures for culturing a cell source which may be found to produce meg-CSF are known to those of skill in the art.

meg-CSF polypeptides may also be produced by known conventional chemical synthesis. Methods for constructing the polypeptides of the present invention by synthetic means are known to those of skill in the art. The synthetically-constructed meg-CSF polypeptide sequences, by virtue of sharing primary, secondary, or tertiary structural and conformational characteristics with neg-CSF polypeptides may possess meg-CSF biological properties in common therewith. Thus, they may be employed as biologically active or immunological substitutes for natural, purified meg-CSF polypeptides in therapeutic and immunological processes.

Other analogs and derivatives of the sequence of meg-CSF which would be expected to retain meg-CSF activity in whole or in part may also be easily made by one of skill in the art given the disclosures herein. One such modification may be the attachment of polyethylene glycol (PEG) onto existing lysine residues in the meg-CSF sequence or the insertion of one or more lysine residues or other amino acid residues that can react with PEG or PEG derivatives into the sequence by conventional techniques to enable the attachment of PEG moieties. Such modifications are believed to be encompassed by this invention.

Thus meg-CSF or active fragments thereof, purified to homogeneity from cell sources or produced synthetically, may be used in a pharmaceutical preparation or formulation to stimulate platelet recovery following chemotherapy or bone marrow transplantation, to treat thrombocytopenia, aplastic anemia and other platelet disorders. Therapeutic treatment of such platelet disorders or deficiencies with these meg-CSF polypeptide compositions may avoid undesirable side effects caused by treatment with presently available serum-derived factors or transfusions of human platelets. It may also be possible to employ one or more peptide fragments of meg-CSF, such as the peptides above-identified, in such pharmaceutical formulations.

The polypeptides of the present invention may also be employed, alone or in combination with other cytokines, hematopoietins, interleukins, growth factors or antibodies in the treatment of the above-identified conditions.

Therefore, as yet another aspect of the invention are therapeutic compositions for treating the conditions referred to above. Such compositions comprise a therapeutically effective amount of the meg-CSF protein or a therapeutically effective fragment thereof in admixture with a pharmaceutically acceptable carrier. This composition can be systematically administered parenterally. Alternatively, the composition may be administered intravenously. If desirable, the composition may be administered subcutaneously. When systematically administered, the therapeutic composition for use in this invention is in the form of a pyrogen-free, parenterally acceptable aqueous solution. The preparation of such pharmaceutically acceptable protein solutions, having due regard to pH, isotonicity, stability and the like, is within the skill of the art.

The dosage regimen involved in a method for treating the above-described conditions will be determined by the attending physician considering various factors which modify the action of drugs, e.g. the condition, body weight, sex and diet of the patient, the severity of any infection, time of administration and other clinical factors. Generally, the daily regimen should be in the range of 1-1000 micrograms of meg-CSF protein or fragment thereof or 50 to 5000 units (i.e., one unit being the minimum concentration of meg-CSF protein which yields the maximal number of colonies in the murine fibrin clot megakaryocyte colony formation assay) of protein per kilogram of body weight.

The compositions and polypeptides of the present invention may also be employed, alone or in combination with other cytokines, hematopoietins, interleukins, growth factors or antibodies in the treatment of disease states characterized by other symptoms as well as platelet deficiencies. It is anticipated that this molecule, if it does not itself have TPO activity, will prove useful in treating some forms of thrombocytopenia in combination with general stimulators of hematopoiesis, such as IL-3, IL-6 or GM-CSF or with other megakaryocytic stimulatory factors or molecules with TPO-like activity. Additional exemplary cytokines or hematopoietins for such co-administration include TPO, G-CSF, CSF-1, GM-CSF, IL-1, IL-11 (described as IL-10 in co-owned copending U. S. patent application SN 07/441,100 incorporated herein by reference), IL-3, IL-4, M-CSF, IL-7 or erythropoietin. The dosage recited above would be adjusted to compensate for such additional components in the therapeutic composition. Progress of the treated patient can be monitored by conventional methods.

Other uses for these novel polypeptides are in the development of antibodies generated by standard methods for in vivo or in vitro diagnostic or therapeutic use. Such antibodies may include both monoclonal and polyclonal antibodies, as well as chimeric antibodies or "recombinant" antibodies generated by known techniques. Also provided by this invention are the cell lines generated by presenting meg-CSF or a fragment thereof as an antigen to a selected mammal, followed by fusing cells of the animal with certain cancer cells to create immortalized cell lines by known techniques. The methods employed to generate such cell lines and antibodies directed against all or portions of a human meg-CSF polypeptide of the present invention are also encompassed by this invention.

The antibodies of the present invention may be utilized for in vivo and in vitro diagnostic purposes, such as by associating the antibodies with detectable labels or label systems. Alternatively these antibodies may be employed for in vivo and in vitro therapeutic purposes, such as by association with certain toxic or therapeutic compounds or moieties known to those of skill in this art. These antibodies also have utility as research reagents.

The following examples illustratively describe the purification and characteristics of homogeneous human meg-CSF and other methods and products of the present invention. These examples are for illustration and do not limit the scope of the present invention.

### Example 1 - Purification of meg-CSF from Urine

The following procedures are presently employed to obtain homogeneous meg-CSF protein from urine of human bone marrow transplant patients. Urine from patients with aplastic anemia or thrombocytopenia accompanying other disease states may also be used as the source of the factor employing this purification.

STEP 1: Urine was collected from the bone marrow transplant patients between days 5 and 18 following transplant. One hundred liters of pooled urine were treated with protease inhibitors phenylmethyl- sulfonylfluoride (PMSF) and ethylenediaminetetraacetic acid (EDTA). This pooled urine was concentrated on an Amicon YM-10 filter (10,000 molecular weight cut-off) to remove excess pigments and reduce the volume. A cocktail of protease inhibitors (leupeptin, aprotinin, ethylene glycol-bis-tetraacetic acid (EGTA) and N-ethylmaleimide (NEM)) was added to the urine at this and the next three steps to minimize proteolysis. The pH of the urine concentrate was adjusted to 8.0 and diluted to a conductivity of 7mS/cm.

STEP 2: The retentate from this first step of the purification was then subjected to anion exchange column chromatography on a QAE Zetaprep [Cuno] at pH 8.0. The QAE flow-through was adjusted to a pH4.5 with lM acetic acid.

STEP 3: The flow-through from the second purification step was bound to a cation exchange chromatographic column, an SP-Zetaprep column [Cuno] at pH 4.5. Bound protein containing meg-CSF was eluted with 1M NaCl at a pH of 4.5. The eluate was pooled, protease inhibitors were added as above and the materials stored at -80°C until further chromatography was performed. The eluate was then dialyzed against Tris-buffered saline (TBS), with the addition of the protease inhibitors described in Step 1. This dialyzate was heated at 56°C for 30 minutes. Addition of the protease inhibitors, while not essential for recovery of protein, enabled greater amount of protein to be recovered from this step, undegraded by the proteases in the system. Pools from this step were also analyzed for the presence of megakaryocyte-specific growth factors. These pools were found to contain meg-CSF activity.

STEP 4: The resulting material was added to a lectin affinity chromatographic column, a Wheat Germ Sepharose column [Pharmacia] and eluted with 0.25 M N-acetyl glucosamine (N-acglcNH₂) in TBS. Urinary meg-CSF was found to bind to this column. The bound protein was eluted from this column by 20 mM sodium acetate, pH 4.5 in the presence of the protease inhibitors of Step 1, which were added for the reasons described in Step 3.

STEP 5: This dialysate was applied to a 10 ml S-Toyopearl FPLC cation exchange column and eluted using a linear gradient of 0 to 1M NaCl in 20mM sodium acetate at pH 4.5. The protein eluted from this step was tested for meg-CSF activity in the fibrin clot assay described below. The meg-CSF activity was observed to elute in two discrete peaks. The major activity eluted between 0.1M and 0.25M NaCl. A minor, but reproducible activity eluted between 0.3M and 0.5N NaCl. The two activities may be due to protein or carbohydrate modification of a single protein; however the data presented further herein refers to the major protein.

STEP 6: The eluate from this fifth purification step was then purified on a reverse phase HPLC (C4) column [Vydac; 1cmX25cm] which was eluted with a linear gradient of between 23-33% acetonitrile in 0.1% trifluoroacetic acid (TFA). This step removes an abundant 30Kd protein contaminant.

STEP 7: The HPLC step was repeated in a different solvent system, after the eluate of Step 6 was diluted with two parts acetic acid and pyridine. The purified material eluted between 6-15% n-propanol in pyridine and acetic acid on a C18 reverse phase HPLC column (0.46 X 25 cm). The material produced after this step, when assayed gave the specific activity of greater than 5 X 10⁷ dilution units reported in the murine assay. This optional step removes the bulk of urinary ribonuclease, a major contaminant, from the preparation.

STEP 8: The HPLC step was repeated once more on a C4 column (Vydac; 0.46 X 25 cm) using 0.15% HFBA in acetonitrile. The material eluted between 27-37% acetonitrile. The last HPLC step removed substantially all remaining ribonuclease and proteinaceous contaminants present after Step 7.

This purified meg-CSF material was then analyzed by SDS-PAGE, bioassayed and labelled with ¹²⁵I. Homogenous protein is obtained from this procedure, omitting step 7, having a specific activity ranging from about 5X10⁷ to about 2-5X10⁸ dilution units per mg protein in the murine megakaryocyte colony assay described below.

### Example 2 - Sodium Dodecyl Sulfate-Polyacrylamide Gel Electrophoresis

The purified protein was analyzed by SDS-PAGE performed according to the method of Laemmli [Laemmli, U. K., Nature, 227:680-685 (1970)] on 12% acrylamide slab gels (0.75 mm thickness). After electrophoresis, the gels were either subjected to autoradiography to visualize ¹²⁵I-labelled meg-CSF, or silver stain, or cut into 0.5-1 cm slices and eluted in 0.5 ml TBS with 0.3% deionized BSA overnight at 4°C and assayed for meg-CSF activity. Apparent molecular weight was determined with protein standards: BRL prestained molecular weight markers, 14C molecular weight standards [NEN], or low molecular weight SDS-PAGE standards [Biorad].

A small aliquot of protein from Steps 6, 7 and 8 of Example 1 containing active meg-CSF was iodinated and subjected to SDS-PAGE. SDS-PAGE analysis (non-reducing conditions) of reverse phase purified meg-CSF from step 8 beginning with several fractions which eluted before the meg-CSF activity, continuing right through the active fractions and ending with fractions which eluted after the peak of meg-CSF activity, revealed the presence of one heterogenous protein band ranging in size between 28 and 38 kd. Elution of the protein from a parallel gel lane revealed that the bioactivity in the murine megakaryocyte colony formation assay correlated with the presence of the iodinated meg-CSF band in the gel.

Upon reduction, the majority of the protein has a molecular weight of between approximately 20-27kd. Based on this information meg-CSF may be a dimer. The protein does not appear to be digestable with N-glycanase under standard conditions.

### Example 3 - Recovery of Protein

Starting with 50 liters of urine, the final pooled active fractions from the HPLC column contained approximately 25 micrograms of protein, estimated from the amino acid composition of purified meg-CSF. The specific activity of the 28-38 kd meg-CSF protein was estimated to be greater than approximately 5 X 10⁷ dilution units/mg in the murine fibrin clot assay described below. One unit of activity is defined as the reciprocal of the maximal dilution which stimulates maximal colony formation. One megakaryocyte colony is defined as 3 or more cells.

### Example 4 - meg-CSF Protein Composition

meg-CSF obtained from the eighth step of the purification of Example 1, omitting Step 7, was employed to obtain tryptic fragments for sequencing. Twenty-five micrograms of purified meg-CSF preparation were desalted over a reverse phase column. The main peak was then fully reduced and alkylated, due to the large number of cysteines present therein. This material was again eluted through a reverse phase column, and the protein peak subjected to SDS-PAGE. Using I¹²⁵ labelled material as a marker, protein running at approximately 20-27 kd was excised from the gel, fixed with methanol:acetic acid:water, and rinsed with water. The gel slice was macerated. Following neutralization with 0.1M NH₄HCO₃ solution (200 µl), the protein contained within the gel matrix was then digested with trypsin (2% w/w).

Sequencing provided the four peptide sequences: (a) Ser Arg Cys Phe Glu Ser Phe Glu Arg (b) Arg Val Cys Thr Ala Glu Leu Ser Cys Lys Gly (Arg) (c) Lys Ala Pro Pro Pro (X) Gly Ala Ser Gln Thr Ile Lys 
(d) Lys Tyr Asp Lys Cys Cys Pro Asp Thr Glu Ser Phe Cys
Ala Glu Val His Asn Pro
 X represents an ambiguously identified amino acid, which is an S or T. () represents a tentatively identified amino acid.

All four of these tryptic peptides are found in the Exons and putative cDNA sequence of meg-CSF.

### Example 5 - Biological Activities of Human meg-CSF

The following assays were performed using the purified meg-CSF described in Example 1.

### A. Murine Fibrin Clot Assay

The meg-CSF obtained from Step 7 of the purification techniques of Example 1 was tested for activity in the megakaryocyte colony formation assay performed substantially as described in S. Kuriya et al, Exp. Hematol., 15:896-901 (1987). A fibrin clot was formed containing 2.5 x 10⁵ mouse bone marrow cells in a 96-well plate. The diluted sample was layered around the clot and incubated for 6 days. Thereafter, cells were fixed and megakaryocytes were stained for acetylcholinesterase, a specific marker for murine megakaryocytes. A colony was defined as three or more megakaryocytes per unit area. Two types of megakaryocyte colonies were routinely observed: pure megakaryocyte colonies containing no additional cell types, and mixed megakaryocyte colonies containing additional non-megakaryocyte cell types.

The following control samples were included in every assay. A positive control was WEHI conditioned medium (murine IL-3), which produced between 7-25 (average 12) megakaryocyte colonies per clot, approximately 50% pure and 50% mixed megakaryocyte colonies. Another positive control was serum taken from lethally irradiated dogs at the nadir of the platelet count [see Mazur et al, Exp. Hematol., 13:1164-1172 (1985)], which produced between 6-22 (average 15) megakaryocyte colonies per clot, of which approximately 70% were pure and 30% were mixed megakaryocyte colonies. The negative control was Iscoves Medium, which produced 2-4 megakaryocyte colonies per clot.

In the assay, the meg-CSF has a specific activity of greater than approximately 5X10⁷ dilution units/mg of protein. A unit of activity is defined as described in Example 3.

The major meg-CSF obtained from bone marrow transplant urine eluted from the S-Toyopearl cation exchange column chromatography step in the purification of Example 1 has been analyzed in this assay alone, together, and in combination with other cytokines. In the fibrin clot assay, it produced between 6-16 (average 13) megakaryocyte colonies, with 50-70% pure megakaryocyte colonies.

In each assay the samples were tested in duplicate and in three dilutions.

### B. Human Plasma Clot meg-CSF Assay

The meg-CSF of this invention was also tested on an assay for human activity, the plasma clot meg-CSF assay described in E. Mazur et al, Blood, 57:277-286 (1981) with modifications. Non-adherent peripheral blood cells were isolated from Leukopacs and frozen in aliquots. The test sample was mixed with platelet-poor human AB plasma and 1.25 x 10⁵ cells in 24-well plates and allowed to clot by the addition of calcium. After a 12 day incubation, megakaryocytes were identified using a monoclonal antibody directed against platelet glycoproteins IIb/IIIa and a horseradish peroxidase/anti-peroxidase chromogenic detection system. Recombinant human IL-3 [Genetics Institute, Inc.] was used as a positive control, producing 12-30 megakaryocyte colonies per clot with approximately 60% pure and 40% mixed megakaryocyte colonies. As in the murine assay, the aplastic dog serum was also used as a positive control, which produced between 5-10 megakaryocyte colonies per clot, of which approximately 50% were pure megakaryocyte colonies contained less than 10 cells, and 50% were mixed megakaryocyte colonies containing more than 40 megakaryocytes. The negative control was Alpha Medium, which produced 0-1 megakaryocyte colonies per clot.

The meg-CSF product from Step 8 of the above-described purification scheme may be active in this assay.

### C. Murine meg-CSF Assay

An assay was performed on the meg-CSF from Step 7 of the purification according to P. J. Quensenberry et al, Blood, 65(1):214-217 (1985). In the assay, the meg-CSF stimulates the growth of acetylcholinesterase positive megakaryocyte colonies containing on average between 4-15 cells per colony. The sizes of the megakaryocytes are variable ranging from small immature cells to morphologically large mature cells.

### D. Other assays

Several additional megakaryocyte assays using murine bone marrow cells were employed including the liquid acetylcholinesterase induction assay of Ishibashi et al, Blood, 69:1737-1741 (1987) and the liquid serotonin uptake assay of Vanucchi et al, exp. Hematol., 16:916-921 (1988).

Fractions were also routinely assayed in several factor dependent cell lines to screen for the presence of growth factors which alone or in combination might stinulate colony formation. The cell lines used were the human erythroleukemic cell line TF-1, the human megakaryoblastic cell line MO-7, the murine Il-6-dependent cell line T1165, and the murine IL-3-dependent cell line DA-1a.

The foregoing descriptions detail presently preferred embodiments of the invention. Numerous modifications and variations in practice of this invention are expected to occur to those skilled in the art. Such modifications and variations are encompassed within the following claims.

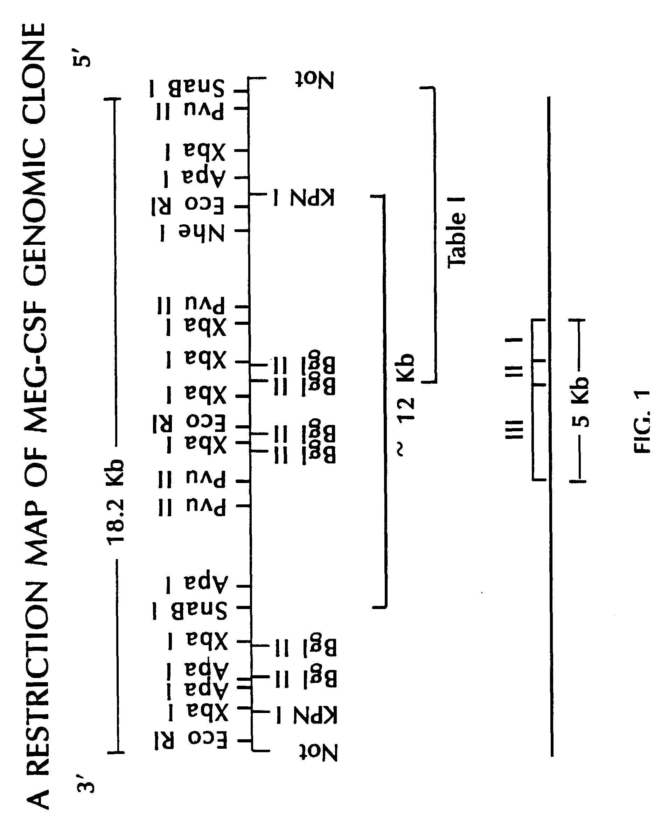

## Claims (Claims for the following Contracting State(s): AT, BE, CH, DE, DK, FR, GB, IT, LI, LU, NL, SE)

1. A megakaryocyte colony stimulating factor protein (meg-CSF), substantially free from association with other proteinaceous material, said protein further characterised by one or more of the following characteristics:
(a) an apparent molecular weight of about 28-38 kD as determined by 12% sodium dodecyl sulfate polyacrylamide gel electrophoresis under non-reducing conditions;
(b) an apparent molecular weight of about 20-27 kD as determined by 12% sodium dodecyl sulfate polyacrylamide gel electrophoresis under reducing conditions; and
(c) the partial amino acid sequences: (i) Ser Arg Cys Phe Glu Ser Phe Glu Arg; 
(ii)    Arg Val Cys Thr Ala Glu Leu Ser Cys Lys
        Gly;
 
(iii)  Lys Ala Pro Pro Pro (X) Gly Ala Ser Gln
       Thr Ile Lys; and
 
(iv)    Lys Tyr Asp Lys Cys Cys Pro Asp Tyr Glu
        Ser Phe Cys Ala Glu Val His Asn Pro,
wherein (X) = Ser or Thr.

2. The protein of claim 1 further characterised by possessing a specific activity of between approximately 5 X 10⁷ and 5 X 10⁸ dilution units/mg protein in a megakaryocyte colony formation assay.

3. The protein according to claim 1 or 2 having the ability to stimulate growth and development of colonies consisting of intermediate and large sized megakaryocyte cells.

4. The protein according to any one of claims 1 to 3 characterised biologically by a specific activity in a murine fibrin clot megakaryocyte colony formation assay of 2 X 10⁸ dilution units/mg protein.

5. The protein according to any one of claims 1 to 4 further characterised by one or more of the following characteristics:
(1) the ability to bind SP-Zeta Prep under acidic conditions of pH4.5;
(2) the ability to bind to Wheat Germ-Sepharose and Concanavalin-A Sepharose;
(3) the ability to elute between 23-33% acetonitrile on a reverse-phase HPLC (C4) column in a solvent of acetonitrile in trifluoroacetic acid;
(4) the ability to elute between 6-15% n-propanol on a reverse-phase HPLC (C18) column in a solvent of n-propanol in pyridine and acetic acid;
(5) the ability to elute between 27-37% acetonitrile on a reverse-phase HPLC (C4) column in a solvent of heptafluorobutyric acid in acetonitrile.

6. The protein according to any one of claims 1 to 5 produced by subjecting urine from human bone marrow transplant patients to purification comprising the steps of:
(a) concentrating said urine;
(b) subjecting the resulting retentate to anion exchange column chromatography;
(c) subjecting the flow-through from step (b) to cation exchange column chromatography;
(d) eluting the material from step (c) through lectin affinity column chromatography;
(e) subjecting the eluate from step (d) to cation exchange fine performance liquid chromatography;
(f) diluting the eluate from step (e) with two parts TFA and subjecting it to reverse phase high pressure liquid chromatography in a solvent of acetonitrile and trifluoroacetic acid;
(g) diluting the eluate from step (f) with two parts pyridine and acetic acid and subjecting it to a second reverse phase high pressure liquid chromatography in a solvent of n-propanol, pyridine and acetic acid; and
(h) optionally subjecting the eluate from step (g) to a third reverse phase high pressure liquid chromatography in a solvent of acetonitrile in heptofluorobutyric acid.

7. A process for preparing homogeneous meg-CSF comprising subjecting urine from bone marrow transplant patients to the purification steps of claim 6, wherein said meg-CSF elutes from the latter column as a single peak.

8. A pharmaceutical composition comprising a therapeutically effective amount of the meg-CSF of claims 1 to 6 or a fragment thereof in a pharmaceutically effective vehicle.

9. The composition according to claim 8 further comprising therapeutically effective amounts of at least one additional cytokine, hematopoietin, growth factor, thrombopoietin-like factor or antibody.

10. The composition according to claim 9 where said cytokine is selected from the group consisting of G-CSF, CSF-1, GM-CSF, IL-1, IL-3, IL-4, erythropoietin, IL-11, IL-6, TPO, M-CSF and IL-7.

11. The composition according to any one of claims 8 to 10 for treating bleeding disorders or platelet deficiencies.

## Claims (Claims for the following Contracting State(s): ES)

1. A process for preparing a megakaryocyte colony stimulating factor protein (meg-CSF), substantially free from association with other proteinaceous material, said protein further characterised by the following characteristics:
(a) an apparent molecular weight of about 28-38 kD as determined by 12% sodium dodecyl sulfate polyacrylamide gel electrophoresis under non-reducing conditions;
(b) an apparent molecular weight of about 20-27 kD as determined by 12% sodium dodecyl sulfate polyacrylamide gel electrophoresis under reducing conditions; and
(c) the partial amino acid sequences: (i)    Ser Arg Cys Phe Glu Ser Phe Glu Arg; 
(ii) Arg Val Cys Thr Ala Glu Leu Ser Cys Lys
Gly;
 
(iii) Lys Ala Pro Pro Pro (X) Gly Ala Ser Gln
Thr Ile Lys; and
 
(iv) Lys Tyr Asp Lys Cys Cys Pro Asp Tyr Glu
Ser Phe Cys Ala Glu Val His Asn Pro,
 wherein (X) = Ser or Thr,
wherein said process comprises subjecting urine from human bone marrow transplant patients to chromatographic purification.

2. The process of claim 1, wherein the protein is further characterised by possessing a specific activity of between approximately 5 X 10⁷ and 5 X 10⁸ dilution units/mg protein in a megakaryocyte colony formation assay.

3. The process according to claim 1 or 2, wherein the protein has the ability to stimulate growth and development of colonies consisting of intermediate and large sized megakaryocyte cells.

4. The process according to any one of claims 1 to 3, wherein the protein is characterised biologically by a specific activity in a murine fibrin clot megakaryocyte colony formation assay of 2 X 10⁸ dilution units/mg protein.

5. The process according to any one of claims 1 to 4, wherein the protein is further characterised by one or more of the following characteristics:
(1) the ability to bind SP-Zeta Prep under acidic conditions of pH4.5;
(2) the ability to bind to Wheat Germ-Sepharose and Concanavalin-A Sepharose;
(3) the ability to elute between 23-33% acetonitrile on a reverse-phase HPLC (C4) column in a solvent of acetonitrile in trifluoroacetic acid;
(4) the ability to elute between 6-15% n-propanol on a reverse-phase HPLC (C18) column in a solvent of n-propanol in pyridine and acetic acid;
(5) the ability to elute between 27-37% acetonitrile on a reverse-phase HPLC (C4) column in a solvent of heptafluorobutyric acid in acetonitrile.

6. The process according to any one of claims 1 to 5 comprising the steps of:
(a) concentrating said urine;
(b) subjecting the resulting retentate to anion exchange column chromatography;
(c) subjecting the flow-throuch from step (b) to cation exchange column chromatography;
(d) eluting the material from step (c) through lectin affinity column chromatography;
(e) subjecting the eluate from step (d) to cation exchange fine performance liquid chromatography;
(f) diluting the eluate from step (e) with two parts TFA and subjecting it to reverse phase high pressure liquid chromatography in a solvent of acetonitrile and trifluoroacetic acid;
(g) diluting the eluate from step (f) with two parts pyridine and acetic acid and subjecting it to a second reverse phase high pressure liquid chromatography in a solvent of n-propanol, pyridine and acetic acid; and
(h) optionally subjecting the eluate from step (g) to a third reverse phase high pressure liquid chromatography in a solvent of acetonitrile in heptofluorobutyric acid.

7. The process of claim 6, wherein said meg-CSF elutes from the latter column as a single peak.

8. A process for the preparation of a pharmaceutical composition comprising combining of a therapeutically effective amount of the meg-CSF of claims 1 to 6 or a fragment thereof with a pharmaceutically effective vehicle.

9. The process according to claim 8, wherein said composition is further combined with therapeutically effective amounts of at least one additional cytokine, hematopoietin, growth factor, thrombopoietin-like factor or antibody.

10. The process according to claim 9 where said cytokine is selected from the group consisting of G-CSF, CSF-1, GM-CSF, IL-1, IL-3, IL-4, erythropoietin, IL-11, IL-6, TPO, M-CSF and IL-7.

11. The process according to any one of claims 8 to 10, wherein said composition is for treating bleeding disorders or platelet deficiencies.

## Patentansprüche (Patentansprüche für folgende(n) Vertragsstaat(en): AT, BE, CH, DE, DK, FR, GB, IT, LI, LU, NL, SE)

1. Megakaryocyten-Koloniestimulierender Proteinfaktor (meg-CSF), der im wesentlichen frei von anderem proteinartigem Material ist, wobei das Protein weiterhin gekennzeichnet ist durch eines oder mehrere der folgenden Merkmale:
(a) ein apparentes Molekulargewicht von etwa 28-38 kD, bestimmt unter nicht-reduzierenden Bedingungen in einer 12 %-Natriumdodecylsulfat-Polyacrylamidgelelektrophorese;
(b) ein apparentes Molekulargewicht von etwa 20-27 kD, bestimmt unter reduzierenden Bedingungen in einer 12 %-Natriumdodecylsulfat-Polyacrylamidgelelektrophorese, und
(c) die partiellen Aminosäuresequenzen: (i)    Ser Arg Cys Phe Glu Ser Phe Glu Arg; (ii)  Arg Val Cys Thr Ala Glu Leu Ser Cys Lys Gly; 
(iii) Lys Ala Pro Pro Pro (X) Gly Ala Ser Gln Thr
Ile Lys; und
 
(iv) Lys Tyr Asp Lys Cys Cys Pro Asp Tyr Glu Ser
Phe Cys Ala Glu Val His Asn Pro,
worin (X) = Ser oder Thr.

2. Protein nach Anspruch 1, das weiterhin dadurch gekennzeichnet ist, daß es eine spezifische Aktivität von zwischen etwa 5 x 10⁷ und 5 x 10⁸ Verdünnungseinheiten/mg Protein in einem Megakaryocyten-Koloniebildungstest besitzt.

3. Protein nach Anspruch 1 oder 2, das die Fähigkeit besitzt, das Wachstum und die Entwicklung von Kolonien zu stimulieren, die aus mittelgroßen und großen Megakaryocyten-Zellen bestehen.

4. Protein nach einem der Ansprüche 1 bis 3, das biologisch durch eine spezifische Aktivität von 2 x 10⁸ Verdünnungseinheiten/mg Protein in einem Megakaryocyten-Koloniebildungstest mit einem Mausfibringerinnsel gekennzeichnet ist.

5. Protein nach einem der Ansprüche 1 bis 4, das weiterhin gekennzeichnet ist durch eines oder mehrere der folgenden Merkmale:
(1) die Fähigkeit, unter sauren Bedingungen von pH 4,5 an SP-Zeta Prep zu binden;
(2) die Fähigkeit, an Weizenkeim-Sepharose und Concanavalin-A-Sepharose zu binden;
(3) die Fähigkeit, in einem Lösungsmittel aus Acetonitril in Trifluoressigsäure zwischen 23-33 % Acetonitril von einer Umkehrphasen-HPLC(C4)-Säule zu eluieren;
(4) die Fähigkeit, in einem Lösungsmittel aus n-Propanol in Pyridin und Essigsäure zwischen 6-15 % n-Propanol von einer Umkehrphasen-HPLC(C18)-Säule zu eluieren;
(5) die Fähigkeit, in einem Lösungsmittel aus Heptafluorbuttersäure in Acetonitril zwischen 27-37 % Acetonitril von einer Umkehrphasen-HPLC(C4)-Säule zu eluieren.

6. Protein nach einem der Ansprüche 1 bis 5, das dadurch hergestellt wird, das Urin von menschlichen Patienten mit einer Knochenmarkstransplantation einer Reinigung unterworfen wird, die folgende Schritte umfaßt:
(a) Konzentrierung des Urins;
(b) Durchführung einer Anionenaustauschsäulenchromatographie mit dem resultierenden Retentat;
(c) Durchführung einer Kationenaustauschsäulenchromatographie mit dem Durchlauf von Schritt (b);
(d) Elution des Materials von Schritt (c) durch Lectin-Affinitätssäulenchromatographie;
(e) Durchführung einer hochauflösenden ("fine performance") Kationenaustausch-Flüssigkeitschromatographie mit dem Eluat von Schritt (d);
(f) Verdünnung des Eluats von Schritt (e) mit zwei Teilen TFA und Durchführung einer Umkehrphasen-Hochdruckflüssigkeitschromatographie mit diesem in einem Lösungsmittel aus Acetonitril und Trifluoressigsäure;
(g) Verdünnung des Eluats von Schritt (f) mit zwei Teilen Pyridin und Essigsäure und Durchführung einer zweiten Umkehrphasen-Hochdruckflüssigkeitschromatographie mit diesem in einem Lösungsmittel aus n-Propanol, Pyridin und Essigsäure; und
(h) gegebenenfalls Durchführung einer dritten Umkehrphasen-Hochdruckflüssigkeitschromatographie in einem Lösungsmittel aus Acetonitril in Heptafluorbuttersäure mit dem Eluat von Schritt (g).

7. Verfahren zur Herstellung eines homogenen meg-CSF, bei dem Urin von Patienten mit einer Knochenmarkstransplantation den Reinigungsschritten nach Anspruch 6 unterworfen wird, wobei der meg-CSF von der letzten Säule als ein einzelner Peak eluiert.

8. Arzneimittel, enthaltend eine therapeutisch wirksame Menge des meg-CSF nach einem der Ansprüche 1 bis 6 oder ein Fragment davon in einem pharmazeutisch wirksamen Vehikel.

9. Arzneimittel nach Anspruch 8, das weiterhin eine therapeutisch wirksame Menge von mindestens einem weiteren Cytokin, Hematopoietin, Wachstumsfaktor, Thrombopoietin-ähnlichem Faktor oder Antikörper enthält.

10. Arzneimittel nach Anspruch 9, worin das Cytokin ausgewählt ist aus G-CSF, CSF-1, GM-CSF, IL-1, IL-3, IL-4, Erythropoietin, IL-11, IL-6, TPO, M-CSF und IL-7.

11. Arzneimittel nach einem der Ansprüche 8 bis 10 zur Behandlung von Blutungsstörungen oder von Mangel an Blutplättchen.

## Patentansprüche (Patentansprüche für folgende(n) Vertragsstaat(en): ES)

1. Verfahren zur Herstellung eines Megakaryocyten-Koloniestimulierenden Proteinfaktors (meg-CSF), der im wesentlichen frei von anderem proteinartigem Material ist, wobei das Protein weiterhin gekennzeichnet ist durch die folgenden Merkmale:
(a) ein apparentes Molekulargewicht von etwa 28-38 kD, bestimmt unter nicht-reduzierenden Bedingungen in einer 12 %-Natriumdodecylsulfat-Polyacrylamidgelelektrophorese;
(b) ein apparentes Molekulargewicht von etwa 20-27 kD, bestimmt unter reduzierenden Bedingungen in einer 12 %-Natriumdodecylsulfat-Polyacrylamidgelelektrophorese, und
(c) die partiellen Aminosäuresequenzen: (i)    Ser Arg Cys Phe Glu Ser Phe Glu Arg; (ii) Arg Val Cys Thr Ala Glu Leu Ser Cys Lys Gly; 
(iii) Lys Ala Pro Pro Pro (X) Gly Ala Ser Gln Thr
Ile Lys; und
 
(iv) Lys Tyr Asp Lys Cys Cys Pro Asp Tyr Glu Ser
Phe Cys Ala Glu Val His Asn Pro,
 worin (X) = Ser oder Thr,
wobei bei dem Verfahren Urin von menschlichen Patienten mit einer Knochenmarkstransplantation einer chromatographischen Reinigung unterworfen wird.

2. Verfahren nach Anspruch 1, wobei das Protein weiterhin dadurch gekennzeichnet ist, daß es eine spezifische Aktivität von zwischen etwa 5 x 10⁷ und 5 x 10⁸ Verdünnungseinheiten/mg Protein in einem Megakaryocyten-Koloniebildungstest besitzt.

3. Verfahren nach Anspruch 1 oder 2, wobei das Protein die Fähigkeit besitzt, das Wachstum und die Entwicklung von Kolonien zu stimulieren, die aus mittelgroßen und großen Megakaryocyten-Zellen bestehen.

4. Verfahren nach einem der Ansprüche 1 bis 3, wobei das Protein biologisch durch eine spezifische Aktivität von 2 x 10⁸ Verdünnungseinheiten/mg Protein in einem Megakaryocyten-Koloniebildungstest mit einem Mausfibringerinnsel gekennzeichnet ist.

5. Verfahren nach einem der Ansprüche 1 bis 4, wobei das Protein weiterhin gekennzeichnet ist durch eines oder mehrere der folgenden Merkmale:
(1) die Fähigkeit, unter sauren Bedingungen von pH 4,5 an SP-Zeta Prep zu binden;
(2) die Fähigkeit, an Weizenkeim-Sepharose und Concanavalin-A-Sepharose zu binden;
(3) die Fähigkeit, in einem Lösungsmittel aus Acetonitril in Trifluoressigsäure zwischen 23-33 % Acetonitril von einer Umkehrphasen-HPLC(C4)-Säule zu eluieren;
(4) die Fähigkeit, in einem Lösungsmittel aus n-Propanol in Pyridin und Essigsäure zwischen 6-15 % n-Propanol von einer Umkehrphasen-HPLC(C18)-Säule zu eluieren;
(5) die Fähigkeit, in einem Lösungsmittel aus Heptafluorbuttersäure in Acetonitril zwischen 27-37 % Acetonitril von einer Umkehrphasen-HPLC(C4)-Säule zu eluieren.

6. Verfahren nach einem der Ansprüche 1 bis 5, das die folgenden Schritte umfaßt:
(a) Konzentrierung des Urins;
(b) Durchführung einer Anionenaustauschsäulenchromatographie mit dem resultierenden Retentat;
(c) Durchführung einer Kationenaustauschsäulenchromatographie mit dem Durchlauf von Schritt (b);
(d) Elution des Materials von Schritt (c) durch Lectin-Affinitätssäulenchromatographie;
(e) Durchführung einer hochauflösenden ("fine performance") Kationenaustausch-Flüssigkeitschromatographie mit dem Eluat von Schritt (d);
(f) Verdünnung des Eluats von Schritt (e) mit zwei Teilen TFA und Durchführung einer Umkehrphasen-Hochdruckflüssigkeitschromatographie mit diesem in einem Lösungsmittel aus Acetonitril und Trifluoressigsäure;
(g) Verdünnung des Eluats von Schritt (f) mit zwei Teilen Pyridin und Essigsäure und Durchführung einer zweiten Umkehrphasen-Hochdruckflüssigkeitschromatographie mit diesem in einem Lösungsmittel aus n-Propanol, Pyridin und Essigsäure; und
(h) gegebenenfalls Durchführung einer dritten Umkehrphasen-Hochdruckflüssigkeitschromatographie in einem Lösungsmittel aus Acetonitril in Heptafluorbuttersäure mit dem Eluat von Schritt (g).

7. Verfahren nach Anspruch 6, wobei der meg-CSF von der letzten Säule als ein einzelner Peak eluiert.

8. Verfahren zur Herstellung eines Arzneimittels, umfassend die Kombination einer therapeutisch wirksamen Menge des meg-CSF nach einem der Ansprüche 1 bis 6 oder eines Fragments davon mit einem pharmazeutisch wirksamen Vehikel.

9. Verfahren nach Anspruch 8, wobei das Arzneimittel weiterhin kombiniert ist mit therapeutisch wirksamen Mengen von mindestens einem weiteren Cytokin, Hematopoietin, Wachstumsfaktor, Thrombopoietin-ähnlichem Faktor oder Antikörper.

10. Verfahren nach Anspruch 9, worin das Cytokin ausgewählt ist aus G-CSF, CSF-1, GM-CSF, IL-1, IL-3, IL-4, Erythropoietin, IL-11, IL-6, TPO, M-CSF und IL-7.

11. Verfahren nach einem der Ansprüche 8 bis 10, wobei das Arzneimittel der Behandlung von Blutungsstörungen oder von Mangel an Blutplättchen dient.

## Revendications (Revendications pour l'(les) Etat(s) contractant(s) suivant(s): AT, BE, CH, DE, DK, FR, GB, IT, LI, LU, NL, SE)

1. Protéine de facteur de stimulation de colonie de mégacaryocytes (meg-CSF), sensiblement exempte d'une association avec une autre matière de type protéique, cette protéine étant en outre caractérisée par une ou plusieurs des caractéristiques suivantes :
(a) un poids moléculaire apparent d'environ 28-38 kD lorsqu'il est déterminé par une électrophorèse sur gel de dodécylsulfatepolyacrylamide sodique 12 %, dans des conditions non réductrices,
(b) un poids moléculaire apparent d'environ 20-27 kD lorsqu'il est déterminé par électrophorèse sur gel de dodécylsulfatepolyacrylamide sodique 12 %, dans des conditions réductrices, et
(c) les séquences partielles d'acides aminés : (i)    Ser Arg Cys Phe Glu Ser Phe Glu Arg, (ii)  Arg Val Cys Thr Ala Glu Leu Ser Cys Lys Gly; 
(iii) Lys Ala Pro Pro Pro (X) Gly Ala Ser Gln Thr
Ile Lys, et
 
(iv) Lys Tyr Asp Lys Cys Cys Pro Asp Tyr Glu Ser
Phe Cys Ala Glu Val His Asn Pro,
où (X) = Ser ou Thr.

2. Protéine suivant la revendication 1, caractérisée en outre en ce qu'elle possède une activité spécifique entre environ 5x10⁷ et 5x10⁸ unités de dilution/mg de protéine dans un essai de formation de colonie de mégacaryocytes.

3. Protéine suivant l'une des revendications 1 et 2, ayant la capacité de stimuler une croissance et un développement de colonies constituées de cellules de mégacaryocytes de taille intermédiaire et de grande taille.

4. Protéine suivant l'une quelconque des revendications 1 à 3, caractérisée biologiquement par une activité spécifique dans un essai de formation de colonie de mégacaryocytes sur coagulation de fibrine murine de 2x10⁸ unités de dilution/mg de protéine.

5. Protéine suivant l'une quelconque des revendications 1 à 4, caractérisée en outre par une ou plusieurs des caractéristiques suivantes :
(1) la capacité de fixer SP-Zeta Prep dans des conditions acides de pH de 4,5;
(2) la capacité de se fixer à du Wheat Germ-Sépharose et à du Concanavaline-A Sépharose;
(3) la capacité d'être élué entre 23-33 % d'acétonitrile sur une colonne de HPLC (C4) à phase inverse dans un solvant d'acétonitrile dans de l'acide trifluoroacétique,
(4) la capacité d'être élué entre 6-15 % de n-propanol sur une colonne de HPLC (C18) à phase inverse dans un solvant de n-propanol dans de la pyridine et de l'acide acétique,
(5) la capacité d'être élué entre 27-37 % d'acétonitrile sur une colonne de HPLC (C4) à phase inverse dans un solvant d'acide heptafluorobutyrique dans de l'acétonitrile.

6. Protéine suivant l'une quelconque des revendications 1 à 5, produite en soumettant de l'urine provenant de patients humains présentant une greffe de moelle osseuse à une purification comprenant les étapes de :
(a) concentration de l'urine,
(b) exposition du produit de rétention résultant à une chromatographie sur une colonne échangeuse d'anions,
(c) exposition de l'écoulement provenant de l'étape (b) à une chromatographie sur une colonne échangeuse de cations,
(d) élution de la matière provenant de l'étape (c) à travers une chromatographie sur colonne par affinité vis-à-vis de la lectine,
(e) exposition du produit d'élution de l'étape (d) à une chromatographie liquide à fine performance par échange de cations,
(f) dilution du produit d'élution de l'étape (e) avec deux parties de TFA et son exposition à une chromatographie liquide à haute pression à phase inverse dans un solvant d'acétonitrile et d'acide trifluoroacétique,
(g) dilution du produit d'élution de l'étape (f) avec deux parties de pyridine et d'acide acétique et son exposition à une deuxième chromatographie liquide à haute pression à phase inverse dans un solvant de n-propanol, pyridine et acide acétique, et
(h) exposition éventuelle du produit d'élution de l'étape (g) à une troisième chromatographie liquide à haute pression à phase inverse dans un solvant d'acétonitrile dans de l'acide heptafluorobutyrique.

7. Procédé de préparation de meg-CSF homogène comprenant une exposition d'urine provenant de patients présentant une greffe de moelle osseuse aux étapes de purification de la revendication 6, dans lequel le meg-CSF est élué à partir de la dernière colonne sous la forme d'un pic unique.

8. Composition pharmaceutique comprenant une quantité thérapeutiquement efficace du meg-CSF suivant l'une des revendications 1 à 6 ou d'un fragment de celui-ci dans un support pharmaceutiquement efficace.

9. Composition suivant la revendication 8, comprenant en outre des quantités thérapeutiquement efficaces d'au moins une cytokine, hématopoïétine, facteur de croissance, facteur de type thrombopoïétine ou anticorps supplémentaire.

10. Composition suivant la revendication 9, dans laquelle ladite cytokine est choisie parmi le groupe comprenant G-CSF, CSF-1, GM-CSF, IL-1, IL-3, IL-4, l'érythropoïétine, IL-11, IL-6, TPO, M-CSF et IL-7.

11. Composition suivant l'une quelconque des revendications 8 à 10, pour le traitement de désordres de saignement ou de déficiences en plaquettes.

## Revendications (Revendications pour l'(les) Etat(s) contractant(s) suivant(s): ES)

1. Procédé de préparation d'une protéine de facteur de stimulation de colonie de mégacaryocytes (meg-CSF), sensiblement exempte d'une association avec une autre matière de type protéique, cette protéine étant en outre caractérisée par une ou plusieurs des caractéristiques suivantes :
(a) un poids moléculaire apparent d'environ 28-38 kD lorsqu'il est déterminé par une électrophorèse sur gel de dodécylsulfatepolyacrylamide sodique 12 %, dans des conditions non réductrices,
(b) un poids moléculaire apparent d'environ 20-27 kD lorsqu'il est déterminé par électrophorèse sur gel de dodécylsulfatepolyacrylamide sodique 12 %, dans des conditions réductrices, et
(c) les séquences partielles d'acides aminés : (i) Ser Arg Cys Phe Glu Ser Phe Glu Arg, (ii)  Arg Val Cys Thr Ala Glu Leu Ser Cys Lys Gly, 
(iii) Lys Ala Pro Pro Pro (X) Gly Ala Ser Gln Thr
Ile Lys, et
 
(iv) Lys Tyr Asp Lys Cys Cys Pro Asp Tyr Glu Ser
Phe Cys Ala Glu Val His Asn Pro,
 où (X) = Ser ou Thr,
ce procédé comprenant une exposition d'urine provenant de patients humains présentant une greffe de moelle osseuse à une purification chromatographique.

2. Procédé suivant la revendication 1, dans lequel la protéine est caractérisée en outre en ce qu'elle possède une activité spécifique entre environ 5x10⁷ et 5x10⁸ unités de dilution/mg de protéine dans un essai de formation de colonie de mégacaryocytes.

3. Procédé suivant l'une des revendications 1 et 2, dans lequel la protéine a la capacité de stimuler une croissance et un développement de colonies constituées de cellules de mégacaryocytes de taille intermédiaire et de grande taille.

4. Procédé suivant l'une quelconque des revendications 1 à 3, dans lequel la protéine est caractérisée biologiquement par une activité spécifique dans un essai de formation de colonie de mégacaryocytes sur coagulation de fibrine murine de 2x10⁸ unités de dilution/mg de protéine.

5. Procédé suivant l'une quelconque des revendications 1 à 4, dans lequel la protéine est en outre caractérisée par une ou plusieurs des caractéristiques suivantes :
(1) la capacité de fixer SP-Zeta Prep dans des conditions acides de pH de 4,5,
(2) la capacité de se fixer à du Wheat Germ-Sépharose et à du Concanavaline-A Sépharose;
(3) la capacité d'être élué entre 23-33 % d'acétonitrile sur une colonne de HPLC (C4) à phase inverse dans un solvant d'acétonitrile dans de l'acide trifluoroacétique,
(4) la capacité d'être élué entre 6-15 % de n-propanol sur une colonne de HPLC (C18) à phase inverse dans un solvant de n-propanol dans de la pyridine et de l'acide acétique,
(5) la capacité d'être élué entre 27-37 % d'acétonitrile sur une colonne de HPLC (C4) à phase inverse dans un solvant d'acide heptafluorobutyrique dans de l'acétonitrile.

6. Procédé suivant l'une quelconque des revendications 1 à 5, comprenant les étapes de :
(a) concentration de l'urine,
(b) exposition du produit de rétention résultant à une chromatographie sur une colonne échangeuse d'anions,
(c) exposition de l'écoulement provenant de l'étape (b) à une chromatographie sur une colonne échangeuse de cations,
(d) élution de la matière provenant de l'étape (c) à travers une chromatographie sur colonne par affinité vis-à-vis de la lectine,
(e) exposition du produit d'élution de l'étape (d) à une chromatographie liquide à fine performance par échange de cations,
(f) dilution du produit d'élution de l'étape (e) avec deux parties de TFA et son exposition à une chromatographie liquide à haute pression à phase inverse dans un solvant d'acétonitrile et d'acide trifluoroacétique,
(g) dilution du produit d'élution de l'étape (f) avec deux parties de pyridine et d'acide acétique et son exposition à une deuxième chromatographie liquide à haute pression à phase inverse dans un solvant de n-propanol, pyridine et acide acétique, et
(h) exposition éventuelle du produit d'élution de l'étape (g) à une troisième chromatographie liquide à haute pression à phase inverse dans un solvant d'acétonitrile dans de l'acide heptafluorobutyrique.

7. Procédé suivant la revendication 6, dans lequel ledit meg-CSF est élué à partir de la dernière colonne sous la forme d'un pic unique.

8. Procédé de préparation d'une composition pharmaceutique comprenant une combinaison d'une quantité thérapeutiquement efficace du meg-CSF suivant l'une des revendications 1 à 6 ou d'un fragment de celui-ci avec un support pharmaceutiquement efficace.

9. Procédé suivant la revendication 8, dans lequel ladite composition est en outre combinée avec des quantités thérapeutiquement efficaces d'au moins une cytokine, hématopoïétine, facteur de croissance, facteur de type thrombopoïétine ou anticorps supplémentaire.

10. Procédé suivant la revendication 9, dans lequel la cytokine est choisie parmi le groupe comprenant G-CSF, CSF-1, GM-CSF, IL-1, IL-3, IL-4, l'érythropoïétine, IL-11, IL-6, TPO, M-CSF et IL-7.

11. Procédé suivant l'une quelconque des revendications 8 à 10, dans lequel ladite composition est prévue pour le traitement de désordres de saignement ou de déficiences en plaquettes.
